(19) Europäisches Patentamt
European Patent Office
Office européen des brevets

(11) Veröffentlichungsnummer: **0 470 434 B1**

(12) **EUROPÄISCHE PATENTSCHRIFT**

(45) Veröffentlichungstag der Patentschrift: **11.10.95**

(51) Int. Cl.[6]: **C07D 251/26**, C07D 251/22, C07D 251/16

(21) Anmeldenummer: **91112373.5**

(22) Anmeldetag: **24.07.91**

Die Akte enthält technische Angaben, die nach dem Eingang der Anmeldung eingereicht wurden und die nicht in dieser Patentschrift enthalten sind.

# (54) Trifluor- bzw. Chlordifluormethoxy-1,3,5,-triazine und Verfahren zu ihrer Herstellung.

(30) Priorität: **03.08.90 DE 4024755**

(43) Veröffentlichungstag der Anmeldung:
**12.02.92 Patentblatt 92/07**

(45) Bekanntmachung des Hinweises auf die Patenterteilung:
**11.10.95 Patentblatt 95/41**

(84) Benannte Vertragsstaaten:
**AT BE CH DE ES FR GB IT LI NL**

(56) Entgegenhaltungen:
**US-A- 3 525 745**

**CHEMICAL ABSTRACTS, , 1964, Columbus, Ohio, US; abstract no. 2986E, MITSUO SUZUKI ET AL.: 'Triazine derivatives'**

**J. A. Chem. Soc. vol. 81, p. 3769-70 (1959)**

(73) Patentinhaber: **BASF Aktiengesellschaft**
**Carl-Bosch-Strasse 38**
**D-67063 Ludwigshafen (DE)**

(72) Erfinder: **Hamprecht, Gerhard, Dr.**
**Rote-Turm-Strasse 28**
**W-6940 Weinheim (DE)**
Erfinder: **Mayer, Horst, Dr.**
**Faselwiese 19**
**W-6700 Ludwigshafen (DE)**
Erfinder: **Wolf, Hans-Josef, Dr.**
**Fichtestrasse 7**
**D-6701 Maxdorf (DE)**

EP 0 470 434 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung teilweise neuer substituierter Trifluor- bzw. Chlordifluormethoxy-1,3,5-triazine der allgemeinen Formel I

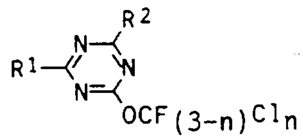

I,

in der $R^1$ und $R^2$ unabhängig voneinander Wasserstoff, ein Halogenatom oder eine Halogenalkylgruppe bedeuten, darüber hinaus $R^1$ auch für einen Trifluor- bzw. Chlordifluormethoxyrest steht und n 0 oder 1 bedeutet und die neuen Endstoffe der Formel I mit Ausnahme des 2,4-Dichlor-6-trifluormethoxy-1,3,5-triazins.

Weiterhin betrifft die vorliegende Erfindung die Verbindungen II und ein Verfahren zu deren Herstellung.

Die Verbindungen I und II dienen als Zwischenprodukte zur Herstellung von Pharmazeutika, Farbstoffen und Pflanzenschutzmitteln, insbesondere zur Herstellung von herbiziden Sulfonylharnstoffderivaten.

Aufgrund der schwierigen Handhabung des äußerst reaktionsfähigen, unselektiven und giftigen Fluors sind bisher keine Methoden zur direkten Fluorierung von Alkoxy-1,3,5-triazinen bekannt geworden. US-A 3 525 745 beschreibt die Herstellung von 2,4-Dichlor-6-trifluormethoxy-1,3,5-triazin durch Umsetzung des giftigen Carbonylfluorids mit Kaliumfluorid in Acetonitril und anschließende Reaktion mit Cyanurchlorid. Aufgrund der niedrigen Ausbeuten (6,1 % d.Th.), der Verwendung eines Autoklaven, der langen Reaktionszeiten von mehr als 20 Stunden pro Reaktionsschritt, der umständlichen Aufarbeitung und der Entsorgung zahlreicher Nebenprodukte ist das bekannte Verfahren nicht wirtschaftlich. In EP-A-70804 wird die Umsetzung des 2-Amino-4-mercapto-6-methoxy-1,3,5-triazins mit Chlordifluormethan zu der entsprechenden 6-Difluormethylthioverbindung beschrieben. Auch hier ist die Ausbeute mit 24 % nicht befriedigend; auch konnte das Verfahren aufgrund der höheren Nukleophilie der Mercaptogruppe gegenüber der Hydroxygruppen nur für die Darstellung von Fluoralkylthio- nicht jedoch von Fluoralkoxy-1,3,5-triazinen genutzt werden. Schließlich erfordert die Handhabung des schädlichen Chlordifluormethans besondere Sicherheitsvorkehrungen, um ein Entweichen in die Atmosphäre zu verhindern.

Der Erfindung lag nun die Aufgabe zugrunde, die erfindungsgemäßen Fluormethoxy-1,3,5-triazine gegenüber dem Stand der Technik auf einfacherem Wege, in kürzerer Reaktionszeit und in besseren Ausbeuten und ohne gleichzeitigen Austausch mehrerer Kernhalogenatome in hoher Selektivität herzustellen.

Demgemäß wurde gefunden, daß man die neuen Trifluor- bzw. Chlordifluormethoxy-1,3,5-triazine der allgmeinen Formel I

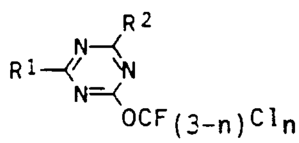

I,

in der $R^1$ und $R^2$ die vorgenannte Bedeutung besitzen, einschließlich dem 2,4-Dichlor-6-trifluormethoxy-1,3,5-triazin, vorteilhaft erhält, wenn man Trichlormethoxy-1,3,5-triazine der Formel II

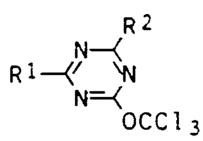

II,

in der $R^1$ und $R^2$ unabhängig voneinander für Wasserstoff, ein Halogenatom oder eine $C_1$-$C_4$-Halogenalkylgruppe, darüber hinaus $R^1$ auch für eine Trichlormethoxygruppe steht, mit Antimontrifluorid, gegebenenfalls in Gegenwart katalytischer Mengen eines Antimon(V)-Salzes, behandelt und den Halogenaustausch bei einer Temperatur von 90 bis 180 °C durchführt.

Die Umsetzung kann für den Fall der Verwendung von 2,4-Difluor-6-trichlormethoxy-1,3,5-triazin und Antimontrifluorid und katalytischer Mengen Antimonpentachlorid durch folgendes Schema beschrieben werden:

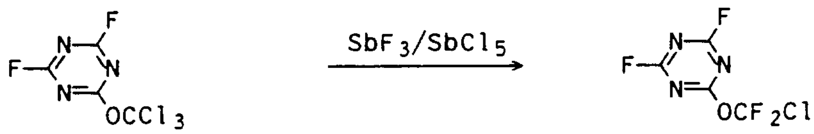


Im Fall der Verwendung von Antimontrifluorid und eines höheren Anteils katalytischer Mengen Antimonpentachlorid läßt sich die Umsetzung durch folgendes Schema wiedergeben:

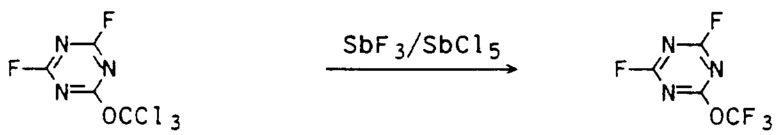


Im Fall der Verwendung von 2,4-Dichlor-6-trichlormethoxy-1,3,5-triazin läßt sich die Umsetzung durch folgendes Schema wiedergeben:

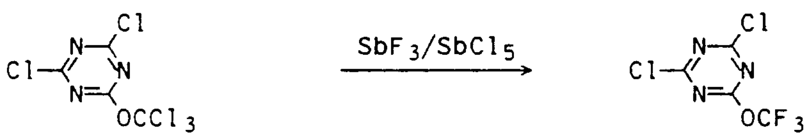


Das Verfahren liefert auf einfachem und wirtschaftlichem Weg neue Trifluormethoxy- bzw. Chlordifluormethoxy-1,3,5-triazine in hoher Ausbeute und Reinheit. Kernständige Chloratome werden hierbei nicht ausgetauscht. Im Hinblick auf den Stand der Technik sind alle diese vorteilhaften Eigenschaften überraschend.

Im Hinblick auf die Weiterverarbeitung zu herbiziden Sulfonylharnstoffderivaten bevorzugte Endstoffe I und dementsprechend bevorzugte Ausgangsstoffe II sind solche, in deren Formeln $R^1$ und $R^2$ unabhängig voneinander Wasserstoff, Fluor, Chlor, Brom, Trichlormethyl, Dichlorfluormethyl, Chlordifluormethyl, Trifluormethyl, 1,1-Dichlor-2,2,2-trifluorethyl, 1,1,2,2,2-Pentafluorethyl, 1,1,2,2,2-Pentachlorethyl bedeuten und darüber hinaus solche Endstoffe I, in denen $R^1$ zusätzlich für einen Trifluormethoxy- bzw. Chlordifluormethoxyrest steht, wenn in den entsprechenden Ausgangsstoffen II $R^1$ eine Trichlormethoxygruppe bedeutet und n für 0 oder 1 steht.

Zweckmäßig verwendet man einen Überschuß von 1 bis 200, vorzugszweise 5 bis 25 Mol-% Antimontrifluorid pro Trichlormethylequivalent. Die Katalysatormenge an Antimon(V)salz beträgt zwischen 1 bis 20, vorzugsweise 5 bis 18 Mol-% pro Trichlormethylequivalent. Vorzugsweise dosiert man den Ausgangsstoff II bei 90 bis 130 °C zu der Mischung des Halogenaustauschmittels und erwärmt dann noch zwischen 10 und ca. 240 Minuten auf eine Temperatur zwischen 110 bis 180 °C. Anschließend wird destillativ aufgearbeitet.

Man kann die Reaktion jedoch auch kontinuierlich führen, den Ausgangsstoff II bei 110 bis 180 °C innerhalb 10 bis ca. 240 Minuten zugeben und gleichzeitig unter vermindertem Druck den entstehenden niedriger siedenden Endstoff I abdestillieren. Spuren mitgerissener Antimonsalze lassen sich durch Extraktion mit konz. Salzsäure beseitigen.

Arbeitet man ohne Antimon(V)salz-Katalyse oder setzt nur geringe Mengen, z.B. 0,5 bis 5 Mol-% ein und reduziert die Menge an Antimontrifluorid auf 60 bis 90 Mol-% pro Trichlormethylequivalent, so bleibt der Halogenaustausch auf der Chlordifluormethoxystufe stehen.

Bevorzugte Endstoffe der Formel I im Hinblick auf deren Weiterverarbeitung zu herbiziden Sulfonylharnstoffderivaten sind beispielsweise 2-Fluor, 4-trifluormethoxy-1,3,5-triazin, 4-Chlor-2-trifluormethoxy-1,3,5-triazin, 2,4-Bis-tri-fluormethoxy-1,3,5-triazin, 2,4-Difluor-6-trifluormethoxy-1,3,5-triazin, 6-Chlordifluormethoxy-2,4-difluor-1,3,5-triazin, 6-Chlordifluormethoxy-2,4-dichlor-1,3,5-triazin, 2-Chlor-4,6-bis-chlordifluormethoxy-1,3,5-triazin, 2, 4-Bis-trifluormethoxy-6-chlor-1,3,5-triazin, 2-Chlor-4-trifluormethoxy-6-trifluormethyl-1,3,5-triazin, 2-Fluor-4-trifluormethoxy-6-trifluormethyl-1,3,5-triazin, 4-Chlordifluormethoxy-2-fluor-6-trifluormethyl-1,3,5-triazin und 2,4-Bis-trifluormethoxy-6-trifluormethyl-1,3,5-triazin.

Die für die Herstellung der fluorierten 1,3,5-Triazine I benötigten Trichlormethoxy-1,3,5-triazine der Formel II

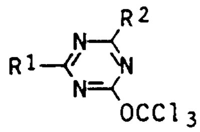

II,

in der $R^1$ und $R^2$ die eingangs erwähnte Bedeutung haben, erhält man vorteilhaft in der Weise, daß man Methoxy-1,3,5-triazine der Formel III,

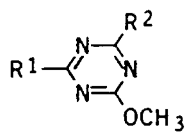

III,

in der $R^1$ und $R^2$ unabhängig voneinander Wasserstoff, ein Fluoratom oder eine $C_1$-$C_4$-Halogenalkylgruppe bedeuten und $R^1$ darüber hinaus auch für eine Methoxygruppe steht, mit einem Chlorierungsmittel bei Temperaturen oberhalb 100 °C behandelt.

Die Chlorierung kann nach einem in der japanischen Offenlegungsschrift 17 039 ('63); Chemical Abstracts 60, 2986 d beschriebenen älteren Verfahren durchgeführt werden, wobei als Lösungsmittel Tetrachlorkohlenstoff verwendet werden muß. Hierbei handelt es sich um ein sehr toxisches Lösungsmittel, dessen Einsatz aus heutiger Sicht der Arbeitssicherheit jedoch zu vermeiden ist (Merkblätter Gefährliche Arbeitsstoffe, Verlag Moderne Industrie, W. Dummer & Co., München, 1977). Tetrachlorkohlenstoff wirkt ferner auch krebserregend (Roth + Daunderer -Giftliste, 8. Erg. Lfg. 12/80; 1979 ecomed Verlagsgesellschaft, Landsberg). Das bekannte Verfahren ist weiterhin in seiner Durchführung und der erforderlichen Reinigungsschritte sehr aufwendig. So muß beispielsweise das 2,4-Dichlor-6-methoxy-1,3,5-triazin einstufig zunächst zu dem 2-Chlormethoxy-4,6-dichlor-1,3,5-triazin chloriert, destilliert und dann durch Umkristallisation gereinigt werden (Beispiel 1). In einem weiteren Verfahrensschritt wird dann zu der Trichlormethoxyverbindung chloriert, wobei erneut destilliert und durch Umkristallisation gereinigt werden muß (Beispiel 6). Es werden nur die Rohausbeuten, nicht jedoch die Ausbeuten nach der Reinigung angegeben. Führt man andererseits die Chlorierung bei hohen Temperaturen durch, so hätte man analog der Chlorierung des 2,4-Dichlor-6-methyl-1,3,5-triazins nur unbefriedigende Ausbeuten an Endstoff, neben der Bildung eines Tetrachlorethans erwartet, das über eine aufwendige Folge von Extraktions-, Destillations-, Kristallisations- und Sublimationsprozessen abgetrennt werden müßte (J. Amer. chem. Soc. 81, 3769 (1959)).

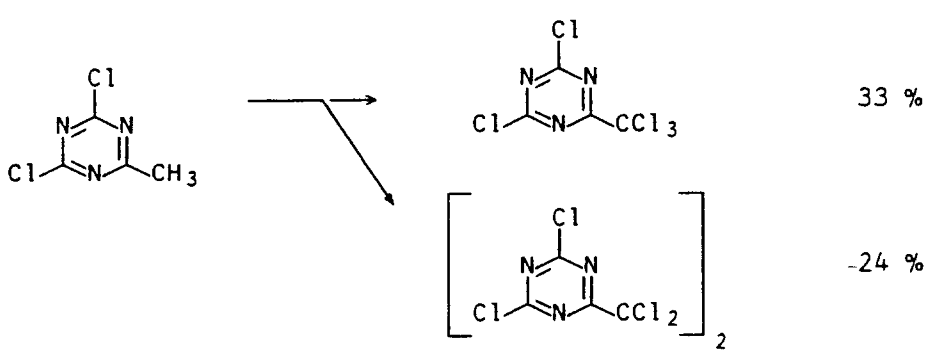


Überraschend wurde nun gefunden, daß man 2,4-Difluor-6-trichlormethoxy-1,3,5-triazine in hohen Ausbeuten, einstufig, ohne störende Nebenreaktionen und durch einfache Aufarbeitung erhalten kann, wenn man die Chlorierung bei höheren Temperaturen, z.B. 100 bis 180 °C durchführt..

Die Umsetzung kann für den Fall der Verwendung von 2,4-Difluor-6-methoxy-1,3,5-triazin und Chlor als Chlorierungsmittel durch folgendes Schema beschrieben werden.

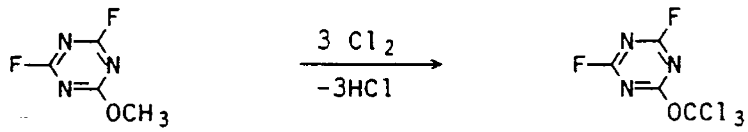


Das Verfahren liefert auf einfachem und wirtschaftlichem Weg neue Trichlormethoxytriazine in hoher Ausbeute und Reinheit.

Bevorzugte Zwischenprodukte II und dementsprechend bevorzugte Ausgangsstoffe III sind solche, in deren Formel $R^1$ und $R^2$ unabhängig voneinander Wasserstoff, Fluor, Trichlormethyl, Dichlorfluormethyl, Chlordifluormethyl, Trifluormethyl, 1,1-Dichlor-2,2,2-trifluorethyl, 1,1,2,2,2-Pentafluorethyl, 1,1,2,2,2-Pentachlorethyl bedeuten und darüber hinaus solche Produkte II, in denen $R^1$ zusätzlich für einen Trichlormethoxyrest steht, wenn in den entsprechenden Ausgangsstoffen $R^1$ eine Methoxygruppe bedeutet.

Als Chlorierungsmittel sind elementares Chlor oder Chlor abgebende Substanzen wie Sulfurylchlorid oder Phosphorpentachlorid geeignet. Chlor kann dabei auch in situ durch Oxydation von Chlorwasserstoffsäure, beispielsweise mit Wasserstoffsuperoxid hergestellt werden.

Die Umsetzung kann in Gegenwart eines inerten höhersiedenden Lösungsmittels, beispielsweise eines chlorierten Kohlenwasserstoffs wie Chlorbenzol, 1,2-, 1,3- oder 1,4-Dichlorbenzol, einer Nitroverbindung wie Nitrobenzol, einer Carbonsäure wie Essigsäure, Propionsäure, einem Säureanhydrid wie Essigsäureanhydrid, einem Säurechlorid wie Chloracetylchlorid, $\alpha$-Chlorpropionsäurechlorid, $\alpha,\alpha$-Dichlorpropionsäurechlorid, einem anorganischen Säurehalogenid wie Phosphortrichlorid oder Phosphoroxychlorid oder bevorzugt ohne Lösungsmittel in der Schmelze des Ausgangsstoffes III durchgeführt werden.

Gegebenenfalls kann man die Reaktion durch Zugabe eines Radikalstarters beschleunigen; als solcher eignet sich die Bestrahlung mit Licht, vorzugsweise UV-Licht oder die Zugabe von $\alpha,\alpha'$-Azoisobutyronitril, zweckmäßig in einer Menge von 0,2 bis 7 Mol-%, bezogen auf den Ausgangsstoff III. Man kann die Reaktion auch durch Zugabe eines Katalysators beschleunigen; als solcher eignet sich Phosphorpentachlorid, zweckmäßig in einer Menge von 0,5 bis 7 Mol-% bezogen auf Ausgangsstoff III. In diesem Fall legt man den Ausgangsstoff zusammen mit dem Katalysator vor und beginnt dann mit der Chlorierung. Statt des Phosphorpentachlorids kann man auch eine dieses unter den Reaktionsbedingungen bildende Ausgangskomponente, z.B. Phosphortrichlorid oder gelben Phosphor, zugeben und dann mit der Chlorierung beginnen.

Ausgangsstoff III kann mit Chlor in annähernd stöchiometrischer Menge oder vorzugsweise im Überschuß, vorteilhaft mit 3,1 bis 11, insbesondere 3,3 bis 5 mol Chlor je Equivalent Methoxy in dem Ausgangsstoff III umgesetzt werden. Die Umsetzung wird bei einer Temperatur von 100 bis 180 °C, vorteilhaft von 120 bis 150 °C, drucklos oder unter Druck, kontinuierlich oder diskontinuierlich, durchgeführt.

Chloriert man bei 1 bar, so werden zweckmäßig 3,3 bis 5 mol Chlorgas, bezogen auf ein Equivalent Methoxy in dem Ausgangsstoff III, eingesetzt, was einem Chlorumsatz von 91 bis 60 % entspricht. Durch geeignete apparative Maßnahmen, z. B. durch Anwendung von mäßigem Überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß.

Die Reaktionszeit beträgt im allgemeinen etwa 0,5 bis 12 Stunden.

In einer bevorzugten Ausführungsform des Verfahrens geht man so vor, daß man innerhalb von 0,5 bis 12 Stunden, vorzugsweise 1 bis 10 Stunden, die erforderliche Menge Chlorgas unter intensivem Rühren in den flüssigen Ausgangsstoff III einleitet, wobei man zunächst bei einer Temperatur von 120 bis 130 °C beginnt und diese - gegebenenfalls unter Ausnutzung des exothermen Charakters der Reaktion -kontinuierlich steigert, so daß gegen Ende die Umsetzung bei einer Temperatur von 135 bis 150 °C durchgeführt wird. Es ist selbstverständlich, daß bei größeren Reaktionsansätzen dem exothermen Charakter durch äußere Kühlung bzw. geeignete Dosierung der Chlormenge Rechnung getragen werden muß; mit dem Abflauen der Reaktion entfernt man das Kältebad und kann gegebenenfalls noch nacherhitzen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in hoher Ausbeute ein bereits recht reines Rohprodukt. Durch Destillation oder Chromatographie kann es weiter gereinigt oder aber direkt für weitere Umsetzungen eingesetzt werden.

Bevorzugte Endstoffe der Formel II sind beispielsweise 2-Fluor, 4-trichlormethoxy-1,3,5-triazin, 4-Chlor-2-trichlormethoxy-1,3,5-triazin, 2,4-Bis-tri-chlormethoxy-1,3,5-triazin, 2,4-Difluor-6-trichlormethoxy-1,3,5-triazin, 2-Fluor-4,6-bis-trichlormethoxy-1,3,5-triazin, 2,4-Dichlor-6-trichlormethoxy-1,3,5-triazin, 2-Chlor-4,6-bis-trichlormethoxy-1,3,5-triazin, 2-Chlor-4-trichlormethoxy-6-trichlormethyl-1,3,5-triazin, 2-Chlor-4-trichlormethoxy-6-trifluormethyl-1,3,5-triazin, 2,4-Bis-trichlormethoxy-6-trichlormethyl-1,3,5-triazin, 2,4-Bis-trichlormethoxy-6-trifluormethyl-1,3,5-triazin, 2-Fluor-4-trichlormethoxy-6-trichlormethyl-1,3,5-triazin, 2-Fluor-4-trichlormethoxy-6-trifluormethyl-1,3,5-triazin.

Die neuen Trichlormethoxy-1,3,5-triazine II sowie die neuen Trifluor- bzw. Chlordifluormethoxy-1,3,5-triazine I sind wertvolle Zwischenprodukte z.B. für die Herstellung von Pflanzenschutzmitteln. Beispielsweise können die Verbindung I z. B. 2,4-Dichlor- oder 2,4-Difluor-6-trifluormethoxy-1,3,5-triazin mit Ammoniak und Methanol in das entsprechende 2-Amino-6-methoxy-4-trifluormethoxy-1,3,5-triazin umgewandelt werden, das mit 2-Carbomethoxy-benzolsulfonylisocyanat zu herbiziden Sulfonylharnstoffen reagiert. Solche Folgeumsetzungen sind in den zeitgleichen Anmeldungen P 40 24 761 (O.Z. 0050/41799) und P 40 24 754 (O.Z. 0050/41800) beschrieben.

Beispiele

I Herstellbeispiel für die Vorprodukte

Beispiel I.1

2,4-Difluor-6-trichlormethoxy-1,3,5-triazin

In eine Mischung von 300 g (2,041 mol) 2,4-Difluor-6-methoxy-1,3,5-triazin und 0,3 g $\alpha,\alpha'$-Azoisobutyronitril leitete man bei 130 °C und unter UV-Bestrahlung einen Strom von Chlorgas so ein, daß sich während 2 Stunden eine Temperatur von 140 bis 145 °C einstellte. Nach NMR-spektroskopischer Kontrolle des Reaktionsverlaufs wurde unter äußerer Beheizung noch weitere 3 Stunden bei 135 bis 140 °C mit Chlor begast. Nach dem Absaugen von ausgefallenem Niederschlag und Destillation des Filtrats im Vakuum erhielt man 444 g (87 % d. Th.) der Titelverbindung vom Sdp. 40 - 46 °C/0,3 mbar.

II Umsetzung zu den Endprodukten I

Beispiel II.1

2,4-Difluor-6-trifluormethoxy-1,3,5-triazin

Zu einer Mischung von 187,4 g (1,048 mol) Antimontrifluorid und 35,2 g (0,117 mol) Antimonpentachlorid wurden die Hälfte von 210 g (0,838 mol) 2,4-Difluor-6-trichlormethoxy-1,3,5-triazin zunächst bei 110 °C unter Rühren so zugegeben, daß sich anfangs eine Temperatur von 125 °C einstellte; mit dem sich einstellenden Rückfluß mußte bei weiterer Zugabe außen beheizt werden. Es wurde eine Stunde bei 125 -130 °C gerührt und eine bei 100 bis 105 °C siedende Fraktion über eine 25-cm-Füllkörperkolonne abdestilliert. Nach dem Abklingen der Reaktion wurde die restliche Hälfte der Trichlormethoxyverbindung innerhalb 30 Minuten zugetropft und die bei 100 bis 105 °C übergehende Fraktion kontinuerliche abdestilliert. Die Gesamtreaktionszeit betrug 3 Stunden. Man erhielt 134,4 g (79,8 % d. Th.) der Titelverbindung mit $n_D^{24}$ = 1.3650.

Beispiel II.2

6-Chlordifluormethoxy-2,4-difluor-1,3,5-triazin

210 g (0,838 mol) 2,4-Difluor-6-trichlormethoxy-1,3,5-triazin wurden innerhalb 10 Minuten unter Rühren bei 110 °C zu 110 g (0,614 mol) Antimontrifluorid gegeben. Nach Zugabe von 3/4 von 9,38 g (0,0313 mol) Antimonpentachlorid wurde auf 145 °C erwärmt und 1 Stunde gerührt. Restlicher Katalysator wurde zugegeben und nochmals 2 Stunden gerührt, wobei als niedersiedende Fraktion über eine 30-cm-Füllkörperkolonne zwischen 95 bis 105 °C 20 g (11,8 % d.Th.) 2,4-Difluor-6-trifluormethoxy-1,3,5-triazin erhalten wurde. Der Destillationsrückstand wurde ohne Kolonne destilliert und ergab 94,8 g (52 % d.Th.) der Titelverbindung vom Sdp. 125 - 130 °C; $n_D^{24}$ = 1.4042.

Beispiel II.3

2,4-Dichlor-6-trifluormethoxy-1,3,5-triazin

Zu einer Mischung von 40,9 g (0,229 mol) Antimontrifluorid und 7,03 g (0,0234 mol) Antimonpentachlorid wurden 52 g (0,183 mol) 2,4-Dichlor-6-trichlormethoxy-1,3,5-triazin innerhalb 5 Minuten unter Rühren bei 90 ° C zugegeben, wobei sich die Temperatur bis auf 180 ° C erhöhte. Es wurde noch 20 Minuten bei 170 bis 180 ° C nachgerührt und dann das Rohprodukt bei 90 - 103 ° C/70 mbar abdestilliert. Durch nochmalige Destillation erhielt man 32,3 g (75,5 % d.Th.) der Titelverbindung vom Sdp. 165 - 173 ° C.

III Umsetzungen der Verbindungen I zu herbizid wirksamen Sulfonylharnstoffderivaten

Beispiel III.1

2-Amino-4-fluor-6-trifiuormethoxy-1,3,5-triazin

4,4 g (0,259 mol) Ammoniakgas wurden innerhalb 45 Minuten bei -70 bis -65 ° C unter Rühren in eine Mischung von 26,0 g (0,1293 mol) 2,4-Difluor-6-trifluormethoxy-1,3,5-triazin in 100 ml Tetrahydrofuran eingeleitet. Es wurde 2 Stunden bei -70 ° C und über Nacht unter Erwärmung bis auf 22 ° C gerührt. Nach dem Einengen im Vakuum wurde der Rückstand mit Wasser verrührt, abgesaugt und gewaschen. Nach dem Trocknen erhielt man 22 g (85,9 % d.Th.) der Titelverbindung vom Fp. 138 - 139 ° C.

Beispiel III.2

2,4-Bismethylamino-6-trifluormethoxy-1,3,5-triazin und 2-Methylamino-4-fluor-6-trifluormethoxy-1,3,5-triazin

5,9 g (0,189 mol) Methylamin wurden bei -70 ° C innerhalb 30 Minuten unter Rühren in eine Mischung von 19,0 g (0,0945 mol) 2,4-Difluor-6-trifluormethoxy-1,3,5-triazin in 100 ml Diethylether eingegast. Es wurde 2 Stunden bei -70 ° C und über Nacht unter Erwärmung bis auf 22 ° C gerührt. Das Reaktionsgemisch wurde im Vakuum eingeengt, in Methylenchlorid aufgenommen und mit Wasser gewaschen. Nach dem Trocknen wurde über eine Kieselgelsäule fraktioniert chromatographiert, wobei man in den ersten beiden Fraktionen 5,0 g (25 % d.Th.) 2-Methylamino-4-fluor-6-trifluormethoxy-1,3,5-triazin vom Fp. 68 -72 ° C erhielt. In den weiteren Fraktionen 4 - 7 isolierte man 10,7 g (51 % d. Th.) des schwerer löslichen 2,4-Bismethylamino-6-trifluormethoxy-1,3,5-triazins vom Fp. 150 - 152 ° C.

Beispiel III.3

2-Amino-4-chlordifluormethoxy-6-fluor-1,3,5-triazin und 2,4-Diamino-6-chlordifluormethoxy-1,3,5-triazin

7,8 g (0,46 mol) Ammoniak wurden innerhalb 45 Minuten unter Rühren bei -70 ° C in eine Mischung von 50,0 g (0,23 mol) 2,4-Difluor-6-chlordifluormethoxy-1,3,5-triazin in 150 ml Tetrahydrofuran eingeleitet. Es wurde 2 Stunden bei -70 ° C und über Nacht unter Erwärmung bis auf 22 ° C gerührt. Das Reaktionsgemisch wurde im Vakuum eingeengt, mit Wasser gewaschen und getrocknet. Anschließend wurde das Reaktionsprodukt mit Methylenchlorid auf eine Kieselgelsäule geschlämmt und mit gleichem Lösungsmittel eluiert. In den Fraktionen 1 bis 8 erhielt man 21,5 g (43,6 % d.Th.) 2-Amino-4-fluor-6-chlordifluormethoxy-1,3,5-triazin vom Fp. 131 - 133 ° C. Durch Nachspülen mit Essigester isolierte man dann in den Fraktionen 9 bis 14 das schwerer lösliche 2,4-Diamino-6-chlordifluormethoxy-1,3,5-triazin (11,2 g, 23 % d.Th.) vom Fp. 114 ° C.

Beispiel III.4

2-Chlordifluormethoxy-4-fluor-6-methylamino-1,3,5-triazin und 2, 4-Bis-methylamino-6-chlordifluormethoxy-1,3,5-triazin

5,2 g (0,166 mol) Methylamin wurden innerhalb 20 Minuten unter Rühren bei -70 ° C in eine Mischung von 18,1 g (0,083 mol) 4-Difluorchlormethoxy-2,6-difluor-1,3,5-triazin eingeleitet. Es wurde 2 Stunden bei -70 ° C und über Nacht unter Erwärmung bis auf 22 ° C gerührt. Das Reaktionsgemisch wurde im Vakuum eingeengt, in Methylenchlorid aufgenommen, mit Wasser gewaschen und getrocknet. Nach Chromatographie über Kieselgel erhielt man in den ersten Fraktionen 5,5 g (29 % d.Th.) 2-Chlordifluormethoxy-4-fluor-6-methylamino-1,3,5-triazin vom Fp. 62 -64 °C. Im Nachlauf isolierte man 8,7 g (44 % d.Th.) 2,4-Bismethylamino-6-chlordifluormethoxy-1,3,5-triazin vom Fp. 118 - 120 °C.

Beispiel III.5

2-Amino-4-methoxy-6-trifluormethoxy-1,3,5-triazin

9,1 g (0,05 mol) 30%iges Natriummethylat wurden bei 0 °C innerhalb 15 Minuten unter Rühren zu einer Mischung von 10 g (0,05 mol) 2-Amino-4-fluor-6-trifluormethoxy-1,3,5-triazin in 100 ml Methanol gegeben. Nach einer Stunde Rühren bei 0 °C wurde im Vakuum eingeengt, in Methylenchlorid aufgenommen und mit Wasser extrahiert. Nach dem Trocknen und Einengen erhielt man 10,5 g (99 % d.Th.) der Titelverbindung vom Fp. 96 - 101 °C.

Beispiel III.6

2-Amino-4-chlordifluormethoxy-6-methoxy-1,3,5-triazin

8,4 g (0,047 mol) 30%iges Natriummethylat wurden bei 0 °C innerhalb 15 Minuten unter Rühren zu einer Mischung von 10 g (0,047 mol) 2-Amino-4-chlordifluormethoxy-6-fluor-1,3,5-triazin in 100 ml Methanol gegeben. Nach einer Stunde Rühren bei 0 °C wurde im Vakuum eingeengt, in Methylenchlorid aufgenommen und mit Wasser extrahiert. Nach dem Trocknen und Einengen erhielt man 10,4 g (98,5 % d. Th.) der Titelverbindung vom Fp. 109 - 110 °C.

Beispiel III.7

2-Amino-4-ethoxy-6-trifluormethoxy-1,3,5-triazin

2,3 g (0,093 mol) 97 % Natriumhydrid wurden portionsweise bei 20 bis 35 °C zu 300 ml Ethanol gegeben und 15 Minuten bis zur Lösung gerührt. Bei 0 °C wurden dann unter Rühren 18,5 g (0,093 mol) 2-Amino-4-fluor-6-trifluormethoxy-1,3,5-triazin innerhalb 10 Minuten zugegeben, 1 Stunde bei 0 °C und über Nacht bei 22 °C gerührt. Nach dem Einengen im Vakuum wurde der Rückstand in Methylenchlorid aufgenommen, mit Wasser extrahiert und getrocknet. Nach dem Einengen erhielt man 17,9 g (85,9 % d.Th.) der Titelverbindung vom Fp. 69 - 71 °C.

Beispiel III.8

2-Amino-4-chlordifluormethoxy-6-ethoxy-1,3,5-triazin

1,2 g (0,047 mol) 97 % Natriumhydrid wurden bei 20 bis 35 °C portionsweise zu 150 ml Ethanol gegeben und 15 Minuten bis zur Lösung gerührt. Anschließend wurde bei 0 °C unter Rühren 10,0 g (0,047 mol) 2-Amino-4-chlordifluormethoxy-6-fluor-1,3,5-triazin zugegeben, 1 Stunde bei 0 °C und über Nacht bei 22 °C gerührt. Nach dem Einengen im Vakuum wurde der Rückstand in Methylenchlorid aufgenommen, mit Wasser extrahiert und getrocknet. Nach dem Einengen erhielt man 10,6 g (94,6 % d.Th.) der Titelverbindung vom Fp. 63 - 65 °C.

Beispiel III.9

2-Amino-4-methylamino-6-trifluormethoxy-1,3,5-triazin

3,5 g (0,111 mol) Methylamin wurden bei 0 °C innerhalb 20 Minuten unter Rühren in eine Lösung von 11 g (0,055 mol) 2-Amino-4-fluor-6-trifluormethoxy-1,3,5-triazin in 150 ml Tetrahydrofuran eingegast. Es wurde eine Stunde bei 0 °C und über Nacht bei 22 °C gerührt. Das Reaktionsgemisch wurde im Vakuum eingeengt, mit Wasser verrührt und getrocknet. Man erhielt 10,8 g (93,1 % d.Th.) der Titelverbindung vom Fp. 155 - 157 °C (Zers.).

Beispiel III.10

2-Amino-4-chlordifluormethoxy-6-methylamino-1,3,5-triazin

2,9 g (0,093 mol) Methylamin wurden innerhalb 20 Minuten unter Rühren bei 0 °C in eine Lösung von 10 g (0,047 mol) 2-Amino-4-chlordifluormethoxy-6-fluor-1,3,5-triazin in 150 ml Diethylether eingegast. Es wurde eine Stunde bei 0 °C und über Nacht bei 22 °C gerührt. Nach dem Waschen mit Wasser, Trocknen und Einengen erhielt man 9,4 g (89,5 % d.Th.) der Titelverbindung vom Fp. 143 °C (Zers.).

Beispiel III.11

2-Amino-4-dimethylamino-6-trifluormethoxy-1,3,5-triazin

5,0 g (0,111 mol) Dimethylamin wurden innerhalb 20 Minuten unter Rühren bei 0 °C in eine Lösung von 11 g (0,055 mol) 2-Amino-4-fluor-6-trifluormethoxy-1,3,5-triazin in 150 ml Tetrahydrofuran eingegast. Es wurde eine Stunde bei 0 °C und über Nacht bei 22 °C gerührt. Nach dem Einengen, Waschen mit Wasser und Trocknen erhielt man 9,9 g (80,7 % d.Th.) der Titelverbindung vom Fp. 114 - 118 °C (Zers.).

Beispiel III.12

2-Amino-4-chlordifluormethoxy-6-dimethylamino-1,3,5-triazin

4,2 g (0,093 mol) Dimethylamin wurden innerhalb 20 Minuten unter Rühren bei 0 °C in eine Lösung von 10 g (0,047 mol) 2-Amino-4-chlordifluormethoxy-6-fluor-1,3,5-triazin in 150 ml Diethylether eingeleitet. Es wurde eine Stunde bei 0 °C und über Nacht bei 22 °C gerührt. Nach dem Waschen mit Wasser, Trocknen und Einengen erhielt man 9,8 g (87,8 % d.Th.) der Titelverbindung vom Fp. 130 - 133 °C (Zers.).

Beispiel III.13

2-(((4-Methoxy-6-trifluormethoxy-1,3,5-triazin-2-yl)aminocarbonyl)-aminosulfonyl)-benzoesäuremethylester

3,6 g (0,015 mol) 2-Carbomethoxy-benzolsulfonylisocyanat in 4 ml 1,2-Dichlorethan wurden innerhalb 5 Minuten unter Rühren bei 22 °C zu einer Mischung von 3,15 g (0,015 mol) 2-Amino-4-methoxy-6-trifluormethoxy-1,3,5-triazin in 150 ml 1,2-Dichlorethan gegeben und 12 Stunden bei 22 °C gerührt. Das Reaktionsgemisch wurde im Vakuum eingeengt und mit Methyltert.-butylether/Petrolether 1 : 1 kristallisiert, abgesaugt und mit Petrolether gewaschen. Man erhielt 5,1 g (75,4 % d. Th.) der Titelverbindung vom Fp. 149 °C (Zers.).

Beispiel III.14

2-(((4-Methoxy-6-trifluormethoxy-1,3,5-triazin-2-yl)aminocarbonyl)-aminosulfonyl)-benzoesäuremethylesternatriumsalz

1,8 g (0,004 mol) der Verbindung aus Beispiel III.13 wurden in 30 ml Methanol suspendiert und mit 0,72 g (0,004 mol) 30%iger Natriummethylatlösung unter Rühren bei 10 - 15 °C versetzt. Die klare Lösung wurde nach 10 Minuten Rühren im Vakuum eingeengt, wobei 1,9 g (100 % d.Th.) der Titelverbindung vom Fp. 118 °C (Zers.) anfielen.

Beispiel III.15

2-(((4-Methylamino-6-trifluormethoxy-1,3,5-triazin-2-yl)amino-carbonyl)aminosulfonyl)-benzoesäureethylester

3,1 g (0,012 mol) 2-Carboethoxy-benzolsulfonylisocyanat in 3 ml Methylenchlorid wurden innerhalb 10 Minuten unter Rühren bei 22 °C zu einer Mischung von 2,5 g (0,012 mol) 2-Amino-4-methylamino-6-trifluormethoxy-1,3,5-triazin in 150 ml Methylenchlorid gegeben und 30 Stunden bei 22 °C gerührt. Das Reaktionsgemisch wurde im Vakuum eingeengt, mit Methyl-tert.-butylether verrührt und abgesaugt. Nach weiterem Waschen mit Methanol und Trocknen erhielt man 3,8 g (67,4 % d. Th.) der Titelverbindung vom Fp. 182 - 184 °C (Zers.).

**Patentansprüche**

**Patentansprüche für folgende Vertragsstaaten : AT, BE, CH, DE, FR, GB, IT, LI, NL**

1. Substituierte Trifluor- bzw. Chlordifluormethoxy-1,3,5-triazine der allgemeinen Formel I

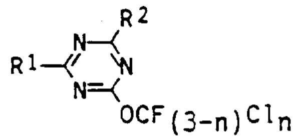

I,

in der $R^1$ und $R^2$ unabhängig voneinander Wasserstoff, ein Halogenatom oder eine $C_1$-$C_4$-Halogenalkylgruppe bedeuten, darüber hinaus $R^1$ auch für einen Trifluor- bzw. Chlordifluormethoxyrest steht und n 0 oder 1 bedeutet, ausgenommen 2,4-Dichlor-6-trifluormethoxy-1,3,5-triazin.

2. Verfahren zur Herstellung von Trifluor- bzw. Chlordifluormethoxy-1,3,5-triazinen der allgemeinen Formel I nach Anspruch 1 einschließlich dem 2,4-Dichlor-6-trifluormethoxy-1,3,5-triazin, dadurch gekennzeichnet, daß man Trichlormethoxy-1,3,5-triazinverbindungen der Formel II

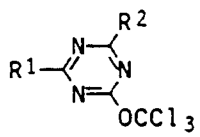

II,

in der $R^1$ und $R^2$ unabhängig voneinander Wasserstoff, ein Halogenatom oder eine $C_1$-$C_4$-Halogenalkylgruppe bedeuten und $R^1$ darüber hinaus auch für eine Trichlormethoxygruppe steht, mit Antimontrifluorid, gegebenenfalls in Gegenwart katalytischer Mengen eines Antimon(V)-salzes, behandelt und den Halogenaustausch bei einer Temperatur von 90 bis 180 °C durchführt.

3. 2,4-Difluor-6-trifluormethoxy-1,3,5-triazin

4. 6-Chlordifluormethoxy-2,4-difluor-1,3,5-triazin

5. Substituierte Trichlormethoxy-triazine der allgemeinen Formel II

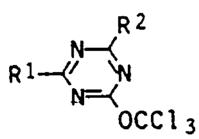

II,

in der $R^1$ und $R^2$ unabhängig voneinander Wasserstoff, ein Fluoratom oder eine $C_1$-$C_4$-Halogenalkylgruppe bedeuten und $R^1$ darüber hinaus auch für eine Trichlormethoxygruppe steht.

6. 2,4-Difluor-6-trichlormethoxy-1,3,5-triazin.

7. Verfahren zur Herstellung von Trifluor- bzw. Chlordifluormethoxy-1,3,5-triazinen der allgemeinen Formel I nach Anspruch 1 einschließlich dem 2,4-Dichlor-6-trifluormethoxy-1,3,5-triazin, dadurch gekennzeichnet, daß man Methoxy-1,3,5-triazinverbindungen der Formel III

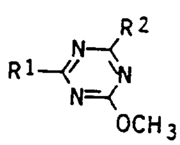

III ,

in der $R^1$ und $R^2$ unabhängig voneinander Wasserstoff, ein Fluoratom oder eine $C_1$-$C_4$-Halogenalkyl-

gruppe bedeuten und $R^1$ darüber hinaus auch für eine Methoxygruppe steht, mit einem Chlorierungsmittel bei Temperaturen oberhalb 100 °C zu Trichlormethoxy-1,3,5-triazinen der allgemeinen Formel II nach Anspruch 5 umsetzt und diese Trichlormethoxytriazine anschließend mit Antimontrifluorid, gegebenenfalls in Gegenwart katalytischer Mengen eines Antimon(V)-salzes behandelt und den Halogenaustausch bei einer Temperatur von 90 bis 180 °C durchführt.

8. Verfahren zur Herstellung von Trichlormethoxy-1,3,5-triazinen der allgemeinen Formel II nach Anspruch 5, dadurch gekennzeichnet, daß man Methoxy-1,3,5-triazinverbindungen der Formel III

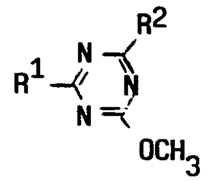

III,

in der $R^1$ und $R^2$ unabhängig voneinander Wasserstoff, ein Fluoratom oder eine $C_1$-$C_4$-Halogenalkylgruppe bedeuten und $R^1$ darüber hinaus auch für eine Methoxygruppe steht, mit einem Chlorierungsmittel bei Temperaturen oberhalb 100 °C behandelt.

**Patentansprüche für folgenden Vertragsstaat : ES**

1. Verfahren zur Herstellung von Trifluor- bzw. Chlordifluormethoxy-1,3,5-triazinen der allgemeinen Formel I

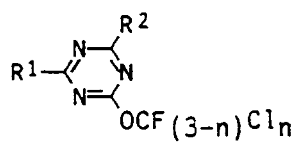

I,

in der $R^1$ und $R^2$ unabhängig voneinander Wasserstoff, ein Halogenatom oder eine $C_1$-$C_4$-Halogenalkylgruppe bedeuten, darüber hinaus $R^1$ auch für einen Trifluor- bzw. Chlordifluormethoxyrest steht und n 0 oder 1 bedeutet, dadurch gekennzeichnet, daß man Trichlormethoxy-1,3,5-triazinverbindungen der Formel II

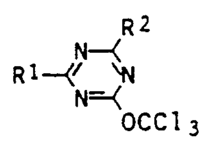

II,

in der $R^1$ und $R^2$ unabhängig voneinander Wasserstoff, ein Halogenatom oder eine $C_1$-$C_4$-Halogenalkylgruppe bedeuten und $R^1$ darüber hinaus auch für eine Trichlormethoxygruppe steht, mit Antimontrifluorid, gegebenenfalls in Gegenwart katalytischer Mengen eines Antimon(V)-salzes, behandelt und den Halogenaustausch bei einer Temperatur von 90 bis 180 °C durchführt.

2. Verfahren zur Herstellung von Trichlormethoxy-1,3,5-triazinen der allgemeinen Formel II

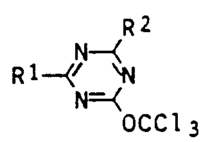

II,

in der $R^1$ und $R^2$ unabhängig voneinander Wasserstoff, ein Fluoratom oder eine $C_1$-$C_4$-Halogenalkyl-

gruppe bedeuten und $R^1$ darüber hinaus auch für eine Trichlormethoxygruppe steht, dadurch gekennzeichnet, daß man Methoxy-1,3,5-triazinverbindungen der Formel III

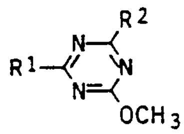

III,

in der $R^1$ und $R^2$ unabhängig voneinander Wasserstoff, ein Fluoratom oder eine $C_1$-$C_4$-Halogenalkylgruppe bedeuten und $R^1$ darüber hinaus auch für eine Methoxygruppe steht, mit einem Chlorierungsmittel bei Temperaturen oberhalb 100 °C behandelt.

**3.** Verfahren nach Anspruch 2, dadurch gekennzeichnet, daß man die Chlorierung mit Chlorgas bei einer Temperatur von 100 bis 180 °C durchführt.

**4.** Verfahren nach den Ansprüchen 2 und 3, dadurch gekennzeichnet, daß man die Chlorierung in Gegenwart von Radikalstartern durchführt.

## Claims

**Claims for the following Contracting States : AT, BE, CH, DE, FR, GB, IT, LI, NL**

**1.** A substituted trifluoro- or chlorodifluoromethoxy-1,3,5-triazine of the formula I

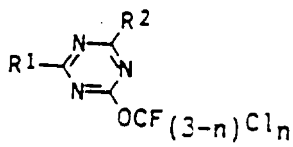

I

where $R^1$ and $R^2$ are each, independently of one another, hydrogen, halogen or $C_1$-$C_4$-haloalkyl, and $R^1$ is also trifluoro- or chlorodifluoromethoxy, and n is 0 or 1, excepting 2,4-dichloro-6-trifluoromethoxy-1,3,5-triazine.

**2.** A process for preparing a trifluoro- or chlorodifluoromethoxy-1,3,5-triazine of the formula I as claimed in claim 1, including 2,4-dichloro-6-trifluoromethoxy-1,3,5-triazine, which comprises treating a trichloromethoxy-1,3,5-triazine of the formula II

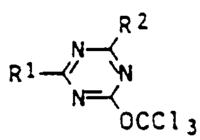

II

where $R^1$ and $R^2$ are each, independently of one another, hydrogen, halogen or $C_1$-$C_4$-haloalkyl, and $R^1$ is also trichloromethoxy, with antimony trifluoride in the presence or absence of a catalytic amount of an antimony(V) salt and carrying out the halogen replacement at from 90 to 180 °C.

**3.** 2,4-Difluoro-6-trifluoromethoxy-1,3,5-triazine.

**4.** 6-Chlorodifluoromethoxy-2,4-difluoro-1,3,5-triazine.

5. A substituted trichloromethoxy-triazine of the formula II

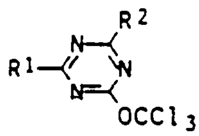

II

where $R^1$ and $R^2$ are each, independently of one another, hydrogen, fluorine or $C_1$-$C_4$-haloalkyl, and $R^1$ is also trichloromethoxy.

6. 2,4-Difluoro-6-trichloromethoxy-1,3,5-triazine.

7. A process for preparing a trifluoro- or chlorodifluoromethoxy-1,3,5-triazine of the formula I as claimed in claim 1, including 2,4-dichloro-6-trifluoromethoxy-1,3,5-triazine, which comprises reacting a methoxy-1,3,5-triazine of the formula III

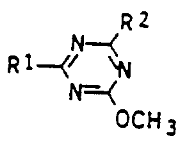

III

where $R^1$ and $R^2$ are each, independently of one another, hydrogen, fluorine or $C_1$-$C_4$-haloalkyl, and $R^1$ is also methoxy, with a chlorinating agent at temperatures above 100 °C to give a trichloromethoxy-1,3,5-triazine of the formula II as claimed in claim 5, subsequently treating this trichloromethoxytriazine with antimony trifluoride in the presence or absence of a catalytic amount of an antimony(V) salt and carrying out the halogen replacement at from 90 to 180 °C.

8. A process for preparing a trichloromethoxy-1,3,5-triazine of the formula II as claimed in claim 5, which comprises treating a methoxy-1,3,5-triazine of the formula III

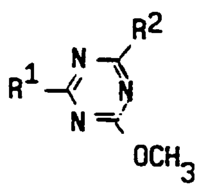

III

where $R^1$ and $R^2$ are each, independently of one another, hydrogen, fluorine or $C_1$-$C_4$-haloalkyl, and $R^1$ is also methoxy, with a chlorinating agent at temperatures above 100 °C.

**Claims for the following Contracting State : ES**

1. A process for preparing a trifluoro- or chlorodifluoromethoxy-1,3,5-triazine of the formula I

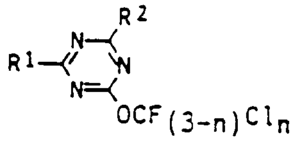

I

where $R^1$ and $R^2$ are each, independently of one another, hydrogen, halogen or $C_1$-$C_4$-haloalkyl, and $R^1$ is also trifluoro- or chlorodifluoromethoxy, and n is 0 or 1, which comprises treating a trichloromethoxy-1,3,5-triazine of the formula II

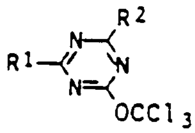

II

where $R^1$ and $R^2$ are each, independently of one another, hydrogen, halogen or $C_1$-$C_4$-haloalkyl, and $R^1$ is also trichloromethoxy, with antimony trifluoride in the presence or absence of a catalytic amount of an antimony(V) salt and carrying out the halogen replacement at from 90 to 180 °C.

**2.** A process for preparing a trichloromethoxy-1,3,5-triazine of the formula II

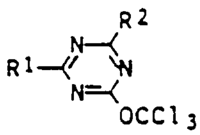

II

where $R^1$ and $R^2$ are each, independently of one another, hydrogen, fluorine or $C_1$-$C_4$-haloalkyl, and $R^1$ is also trichloromethoxy, which comprises treating a methoxy1,3,5-triazine of the formula III

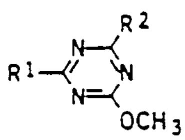

III

where $R^1$ and $R^2$ are each, independently of one another, hydrogen, fluorine or $C_1$-$C_4$-haloalkyl, and $R^1$ is also methoxy, with a chlorinating agent at temperatures above 100 °C.

**3.** A process as claimed in claim 2, wherein the chlorination is carried out with gaseous chlorine at from 100 to 180 °C.

**4.** A process as claimed in claims 2 and 3, wherein the chlorination is carried out in the presence of radical initiators.

**Revendications**
**Revendications pour les Etats contractants suivants : AT, BE, CH, DE, FR, GB, IT, LI, NL**

**1.** Trifluoro- et chlorodifluoro-méthoxy-1,3,5-triazines substituées de formule générale I

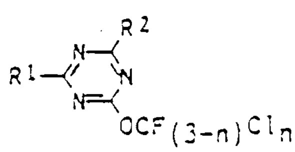

I,

dans laquelle $R^1$ et $R^2$ représentent chacun, indépendamment l'un de l'autre, l'hydrogène, un atome d'halogène ou un groupe halogénoalkyle en C1-C4, $R^1$ pouvant en outre représenter un groupe trifluoro- ou chlorodifluoro-méthoxy, et n est égal à 0 ou 1, à l'exception de la 2,4-dichloro-6-trifluorométhoxy-1,3,5-triazine.

**2.** Procédé de préparation des trifluoro- et chlorodifluoro-méthoxy-1,3,5-triazines de formule générale I de la revendication 1, y compris de la 2,4-dichloro-6-trifluoro-méthoxy-1,3,5-triazine,
caractérisé en ce que l'on traite des dérivés de la trichlorométhoxy-1,3,5-triazine répondant à la formule II

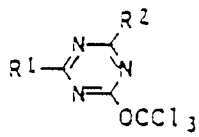

II,

dans laquelle $R^1$ et $R^2$ représentent chacun, indépendamment l'un de l'autre, l'hydrogène, un atome d'halogène ou un groupe halogénoalkyle en C1-C4, $R^1$ pouvant en outre représenter un groupe trichlorométhoxy, par le trifluorure d'antimoine, éventuellement en présence de quantités catalytiques d'un sel d'antimoine-V, et on procède à l'échange d'halogène à une température de 90 à 180°C.

3. La 2,4-difluoro-6-trifluorométhoxy-1,3,5-triazine.

4. La 6-chlorodifluorométhoxy-2,4-difluoro-1,3,5-triazine.

5. Trichlorométhoxytriazines substituées de formule générale II

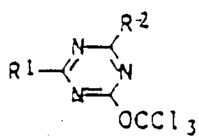

II,

dans laquelle $R^1$ et $R^2$ représentent chacun, indépendamment l'un de l'autre, un atome de fluor ou un groupe halogénoalkyle en C1-C4, $R^1$ pouvant en outre représenter un groupe trichlorométhoxy.

6. La 2,4-difluoro-6-trichlorométhoxy-1,3,5-triazine.

7. Procédé de préparation des trifluoro- et chlorodifluoro-méthoxy-1,3,5-triazines de formule générale I de la revendication 1, y compris de la 2,4-dichloro-6-trifluorométhoxyl-1,3,5-triazine, caractérisé en ce que l'on fait réagir des dérivés de la méthoxy-1,3,5-triazine répondant à la formule III

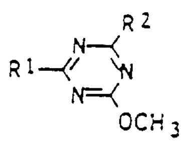

III,

dans laquelle $R^1$ et $R^2$ représentent chacun, indépendamment l'un de l'autre, l'hydrogène, un atome de fluor ou un groupe halogénoalkyle en C1-C4, $R^1$ pouvant en outre représenter un groupe méthoxy, par un agent chlorant à des températures supérieures à 100°C, ce qui donne les trichlorométhoxy-1,3,5-triazines de formule générale II de la revendication 5 qu'on traite ensuite par le trifluorure d'antimoine, éventuellement en présence de quantités catalytiques d'un sel d'antimoine-V et on procède à l'échange d'halogène à une température de 90 à 180°C.

8. Procédé de préparation des trichlorométhoxy-1,3,5-triazines de formule générale II de la revendication 5, caractérisé en ce que l'on traite des dérivés de la méthoxy-1,3,5-triazine répondant à la formule III

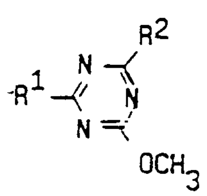

III,

dans laquelle $R^1$ et $R^2$ représentent chacun, indépendamment l'un de l'autre, l'hydrogène, un atome de fluor ou un groupe halogénoalkyle en C1-C4, $R^1$ pouvant en outre représenter un groupe méthoxy, par un agent chlorant à des températures supérieures à 100 ° C.

**Revendications pour l'Etat contractant suivant : ES**

**1.** Procédé de préparation des trifluoro- et chlorodifluoro-méthoxy-1,3,5-triazines de formule générale I

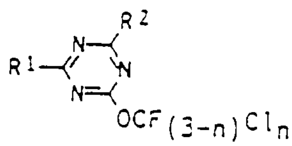

I,

dans laquelle $R^1$ et $R^2$ représentent chacun, indépendamment l'un de l'autre, l'hydrogène, un atome d'halogène ou un groupe halogénoalkyle en C1-C4, $R^1$ pouvant en outre représenter un groupe trifluoro- ou chlorodifluoro-méthoxy et n est égal à 0 ou 1, caractérisé en ce que l'on traite des dérivés de la trichlorométhoxy-1,3,5-triazine répondant à la formule II

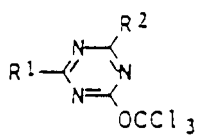

II,

dans laquelle $R^1$ et $R^2$ représentent chacun, indépendamment l'un de l'autre, l'hydrogène, un atome d'halogène ou un groupe halogénoalkyle en C1-C4, $R^1$ pouvant en outre représenter un groupe trichlorométhoxy, par le trifluorure d'antimoine, éventuellement en présence de quantités catalytiques d'un sel d'antimoine-V, et on procède à l'échange d'halogène à une température de 90 à 180 ° C.

**2.** Procédé de préparation des trichlorométhoxy-1,3,5-triazines de formule générale II

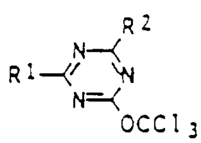

II,

dans laquelle $R^1$ et $R^2$ représentent chacun, indépendamment l'un de l'autre, l'hydrogène, un atome de fluor ou un groupe halogénoalkyle en C1-C4, $R^1$ pouvant en outre représenter un groupe trichlorométhoxy, caractérisé en ce que l'on traite des dérivés de la méthoxy-1,3,5-triazine répondant à la formule III

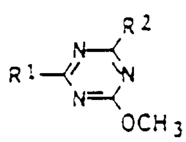

III,

dans laquelle $R^1$ et $R^2$ représentent chacun, indépendamment l'un de l'autre, l'hydrogène, un atome de fluor ou un groupe halogénoalkyle en C1-C4, $R^1$ pouvant en outre représenter un groupe méthoxy, par un agent chlorant à des températures supérieures à 100 ° C.

**3.** Procédé selon la revendication 2, caractérisé en ce que l'on traite à l'aide du chlore gazeux à une température de 100 à 180 ° C.

4. Procédé selon les revendications 2 et 3, caractérisé en ce que l'on procède à la chloruration en présence d'inducteurs radicalaires.